# EUROPEAN PATENT SPECIFICATION

(11) **EP 4 631 564 B1**
(45) Date of publication and mention of the grant of the patent: **09.09.2026**
(21) Application number: 25169939.3
(22) Date of filing: 11.04.2025
(51) Int. Cl.: A61N 1/372, A61N 1/375, A61N 1/362, A61N 1/05

(54) **BIOSTIMULATOR TRANSPORT SYSTEM HAVING TETHERING MECHANISM**
BIOSTIMULATORTRANSPORTSYSTEM MIT HALTEMECHANISMUS
SYSTÈME DE TRANSPORT DE BIOSTIMULATEUR AYANT UN MÉCANISME D'ATTACHE

(30) Priority: 12.04.2024 US 202463633432 P; 08.04.2025 US 202519173722
(43) Date of publication of application: 15.10.2025
(73) Proprietor: Pacesetter, Inc., Sylmar, CA 91342 (US)
(72) Inventor: BLUE, Jeremiah, Sylmar, CA 91342 (US)
(74) Representative: Barker Brettell Sweden AB

(56) References cited:
- WO-A2-2008/021969
- CN-A- 101 641 061
- US-A1- 2022 409 885
- US-A1- 2024 001 112

## Description

### TECHNICAL FIELD

The disclosed embodiments relate generally to biostimulators. More specifically, but not exclusively, the disclosed embodiments relate to transport systems used to deliver or retrieve biostimulators, such as leadless pacemakers.

### BACKGROUND INFORMATION

Cardiac pacing by an artificial pacemaker, known more generally as a "biostimulator," provides electrical stimulation of the heart when the natural pacemaker and/or conduction system of the heart fails to provide synchronized atrial and ventricular contractions at healthy rates and intervals. Such antibradycardial pacing provides relief from symptoms, and even life support, for hundreds of thousands of patients. Cardiac pacing can also provide electrical overdrive stimulation to suppress or convert tachyarrhythmias, again supplying relief from symptoms and preventing or terminating arrhythmias that could lead to sudden cardiac death.

Currently available or conventional pacemakers usually perform cardiac pacing using an electrical pulse generator implanted subcutaneously or sub-muscularly in or near a pectoral region of a patient. Operating parameters of the pulse generator are usually interrogated and modified in one of three ways: by a programming device outside the body, via a loosely-coupled transformer with one inductance inside the body and another outside, or via electromagnetic radiation with one antenna inside the body and another outside. The pulse generator usually connects to the proximal end of one or more implanted leads, the distal ends of which contain one or more electrodes for positioning adjacent to the inside or outside wall of a cardiac chamber. The leads have an electrical conductor for connecting the pulse generator to electrodes in the heart. Such electrode leads typically have lengths of 50-70 centimeters. Pacing leads can engage and/or be fixed to an intracardial implant site by an engaging mechanism such as an electrode anchor. For example, the electrode anchor can screw into the myocardium.

Leadless cardiac pacemakers locate electronic circuitry at the pacing site, thereby eliminating electrical leads and avoiding shortcomings associated with conventional cardiac pacing systems. Leadless cardiac pacemakers can be anchored at a pacing site-for instance, in a right ventricle for single-chamber pacing and in both a right ventricle and a right atrium for dual-chamber pacing - by an anchor. A delivery system can be used to deliver the leadless cardiac pacemakers to the target anatomy and a recovery system, which in some instances can be the same as the delivery system, is used to retrieve a leadless pacemaker.

US 2024/001112 A1 discloses a biostimulator transport system including a flexible tether extending through a tether support to a tether bight. The tether bight loops through an attachment feature of a biostimulator to connect the biostimulator to the biostimulator transport system. Tether legs extend from the tether bight to a handle of the biostimulator transport system. The handle includes a housing and a knob, and the tether legs attach to the housing and the knob. A tether leg can be disconnected from the housing and the knob can be removed to pull the tether out of the attachment feature and release the biostimulator from the biostimulator transport system.

### SUMMARY

Existing delivery systems used to implant leadless pacemakers can include rigid tether systems. For example, solid wires can engage and connect to a leadless pacemaker to retain the leadless pacemaker relative to the delivery system. The wires may, however, fail under cyclical stress applied by a beating heart. Accordingly, delivery systems that can reliably transport leadless pacemakers to an implant site with reduced risk of failure are desirable.

The present invention is defined in the independent claim. Further embodiments of the invention are defined in the dependent claims.

### BRIEF DESCRIPTION OF THE DRAWINGS

Non-limiting and non-exhaustive embodiments of the present invention are described with reference to the following figures, in which like reference numerals refer to like parts throughout the various views unless otherwise specified.
FIG. 1 is a diagrammatic cross section of a human heart illustrating an implantation of a biostimulator in a target anatomy, in accordance with an embodiment.
FIG. 2 is a perspective view of a biostimulator system including a biostimulator and a biostimulator transport system, in accordance with an embodiment.
FIGS. 3A-3B are a perspective view and a side cross-section, respectively, of a tethering apparatus for a biostimulator transport system, in accordance with an embodiment.
FIGS. 3C-3D are perspective views of an operation of the tethering apparatus of FIGS. 3A-3B, in accordance with an embodiment.
FIGS. 4A-4D are perspective views of a connection between a biostimulator, a tether, and a tethering mechanism of a biostimulator transport system, in accordance with an embodiment.
FIG. 5 is a plan view of a tether, in accordance with an embodiment.
FIGS. 6A-6C are side views of operation of a tether and a tethering apparatus of a biostimulator transport system, in accordance with an embodiment.
FIGS. 7A-7B are cross-sectional side views of operation of a tethering apparatus of a biostimulator transport system, in accordance with an embodiment.

### DETAILED DESCRIPTION

Embodiments are described of a system for delivery of biostimulators such as leadless pacemakers. Specific details are described to provide an understanding of the embodiments, but one skilled in the relevant art will recognize that the invention can be practiced without one or more of the described details, components, materials, etc. In some instances, well-known structures, materials, or operations are not shown or described in detail but are nonetheless encompassed within the scope of the invention.

Reference throughout this specification to "one embodiment" or "an embodiment" means that a described feature, structure, or characteristic can be included in at least one described embodiment, so that appearances of "in one embodiment" or "in an embodiment" do not necessarily all refer to the same embodiment. Furthermore, the described features, structures, or characteristics can be combined in any suitable manner in one or more embodiments.

As used in this application, directional terms such as "left," "right," "front," "rear," "upper," lower," "top," "bottom," "side," "lateral," "longitudinal," etc., refer to the orientations of embodiments as they are presented in the drawings, but any directional term should not be interpreted to imply or require a particular orientation of the described embodiments when in actual use. The use of relative terms throughout the description can denote a relative position or direction. For example, "distal" can indicate a first direction along a longitudinal axis of a biostimulator transport system and "proximal" can indicate a second direction opposite to the first direction. Such terms are provided to establish relative frames of reference, but are not intended to limit the use or orientation of a biostimulator transport system to a specific configuration described in the various embodiments below.

Implanting a biostimulator such, as a leadless pacemaker, in a heart of a patient is accomplished using a biostimulator transport system, e.g., a delivery system, - usually, but not necessarily, a catheter. Implantation of the biostimulator is typically a process including one or more of: preparation, insertion and implantation, tether mode, release, and catheter removal. These operations are described further below.

**Preparation.** One end of the biostimulator (also known as the "button" or the "attachment feature" of the biostimulator) is attached to a flexible tether and the tether is pulled into an interior of the catheter until the button is engaged by an end cap of the catheter (also known as a "docking cap"). The end cap is positioned at a distal end of the catheter and includes a socket that can engage and release the biostimulator and that allows the catheter to exert forces and torques on the biostimulator during implantation.

**Insertion and Implantation.** The biostimulator, now attached to the distal end of the catheter, is inserted in a body of the patient and transported by the catheter through the body to the specific heart tissue where it will be implanted. Once there, the attachment mechanism of the biostimulator is used to attach the biostimulator to the heart tissue. In one embodiment, fixation to the heart tissue is achieved by mechanical means such as a screw-in helical screw or tines (small hooks extending from the biostimulator).

**Tether Mode.** After the biostimulator is attached to the heart tissue, the biostimulator is released from the end cap of the catheter but remains attached to the flexible tether. The purpose of this "tether mode" is to evaluate the attachment of the biostimulator to the heart while retaining the ability to retrieve the biostimulator by pulling it back into engagement with the end cap if biostimulator attachment proves unsatisfactory. Because the tether is flexible, tether mode allows the biostimulator to move along with the heart as it beats, simulating the loads and movements to which the biostimulator will be subjected when the implantation is complete.

**Biostimulator Release and Catheter Removal.** If the attachment of the biostimulator to the heart tissue is satisfactory, then the flexible tether is detached from the button of the biostimulator and the tether is partially or fully pulled into an interior of the catheter until it is no longer attached to the biostimulator, leaving the biostimulator in the heart. The catheter, now without the biostimulator on its end, is transported back out of the patient.

**Repeat.** In a case where multiple biostimulators will be implanted in the heart of the patient, some or all of the steps above can be repeated. Otherwise, the procedure is substantially complete.

The embodiments disclosed and discussed below are directed to improving this process.

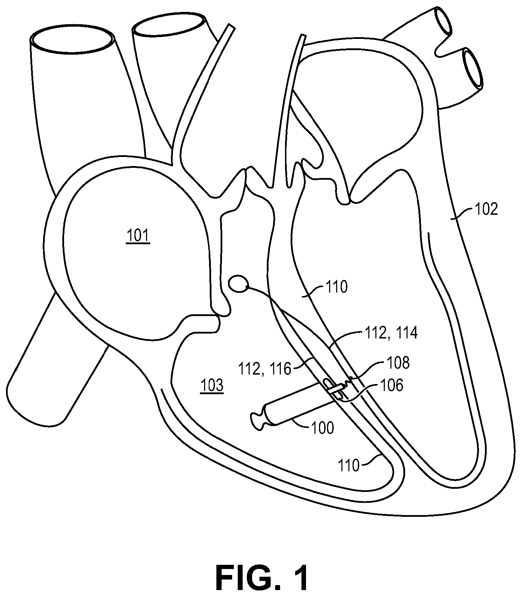

FIG. 1 illustrates an embodiment of implantation of a biostimulator in a target anatomy of a human heart. A leadless biostimulator system, e.g., a cardiac pacing system, includes one or more biostimulators 100. Biostimulator 100 can be implanted in a patient heart 102, and can be leadless (e.g., it can be a leadless pacemaker). Each biostimulator 100 can be placed in a cardiac chamber, such as a right atrium 101 and/or right ventricle 103 of heart 102, or attached to an inside or outside of the cardiac chamber. For example, biostimulator 100 can be attached to a septum 110 of heart 102. More particularly, biostimulator 100 can be delivered to the septum 110, and one or more elements, such as a fixation element 106 and/or a pacing element 108, can pierce a septal wall 110 to engage and anchor the biostimulator 100 to the target anatomy, e.g., a bundle branch 112 in the septal wall 110. In a particular embodiment, biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

When biostimulator 100 is delivered to and screwed into the target tissue, e.g., an atrial or ventricular wall, or a septum 110 of the heart 102, pacing element 108 and/or fixation element 106 can be positioned for pacing. For example, in the case of deep septal pacing at respective bundle branches 112 in septum 110, an active electrode of pacing element 108 can be positioned at a first target anatomy in the septal wall 110, e.g., a left bundle branch 114. Similarly, the fixation element 106 can be positioned at a second target anatomy in the septal wall, e.g., a right bundle branch 116. Optionally, one of the elements may be at a bundle branch and the other element may not be at a bundle branch.

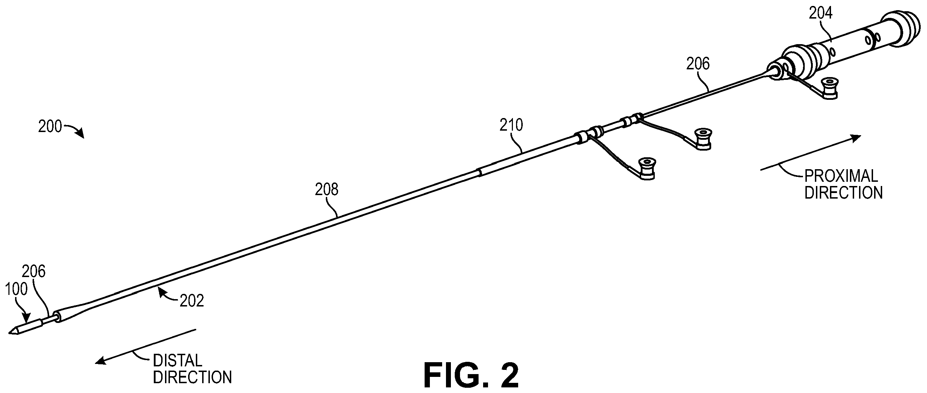

FIG. 2 illustrates an embodiment of a biostimulator system 200. Biostimulator system 200 can include a biostimulator 100, e.g., a leadless pacemaker or other leadless biostimulator. System 200 can also include a biostimulator transport system 202, e.g., delivery or retrieval systems, which may be catheter-based systems used to carry biostimulator 100 intravenously to or from a patient anatomy. For example, a biostimulator transport system 202 can be used to deliver biostimulator 100 to, or retrieve the biostimulator from, a patient. Biostimulator 100 can be attached, connected to, or otherwise mounted on biostimulator transport system 202. The biostimulator can be mounted on a distal end of a catheter of the biostimulator transport system 202, which catheter is then used to advance biostimulator 100 intravenously into or out of heart 102.

Biostimulator transport system 202 can include a handle 204 to control movement and operations of the transport system from outside a patient. One or more elongated members extend distally from handle 204. For example, an elongated catheter body 206 can extend distally from handle 204 to a distal end of biostimulator transport system 202. In an embodiment, biostimulator 100 is mounted on a distal end of elongated catheter body 206.

Biostimulator transport system 202 can include a protective sleeve 208 to cover biostimulator 100 during delivery and implantation. Protective sleeve 208 can extend over, and be longitudinally movable relative to, elongated catheter body 206. The biostimulator transport system can also include an introducer sheath 210 that can extend over, and be longitudinally movable relative to, the protective sleeve 208. Introducer sheath 210 can cover a distal end of the protective sleeve 208, the elongated catheter body 206, and biostimulator 100, as those components are passed through an access device into the patient anatomy.

Several components of biostimulator transport system 202 are described above, but the biostimulator transport system can be configured to include additional or alternate components. More particularly, biostimulator transport system 202 can be configured to deliver biostimulator 100 to, or retrieve it from, the target anatomy. Delivery and/or retrieval of biostimulator 100 can include retaining the biostimulator 100 on biostimulator transport system 202 during transport to the target anatomy and rotation of the biostimulator during its implantation at the target anatomy. Accordingly, biostimulator transport system 202 can incorporate features to retain and rotate biostimulator 100.

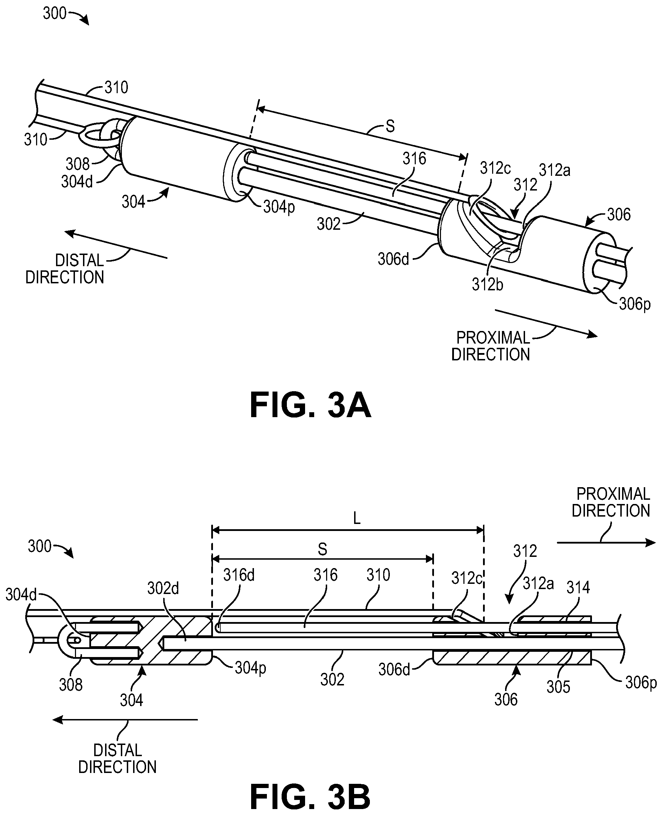

FIGS. 3A-3B together illustrate an embodiment of a tethering apparatus 300. Tethering apparatus 300 includes a support wire 302 with a distal end 302d. The support wire also has a proximal end that is not shown in these drawings but could, in some embodiments, be located at or near the proximal end of a catheter such as the one shown in FIG. 2. Two primary components are attached to the support wire: a distal anchor 304 attached to the distal end 302d, and a proximal anchor 306 also attached to support wire 302 but proximally spaced apart (i.e., spaced apart in a proximal direction of the support wire) by a distance S from the distal anchor. The proximal spacing creates a gap between the proximal and distal anchors. In one embodiment, distance S can range from 0.1 inches to 0.25 inches, but in other embodiments distance S can be below or above this range.

Distal anchor 304 has a distal end 304d and a proximal end 304p, and distal end 302d of the support wire is inserted through proximal end 304p into a hole in the distal anchor. Once inserted, the support wire is fixed in place so that distal anchor 304 is attached to distal end 302d and cannot move relative to the support wire. A tether attachment 308 is also attached to distal end 304d to hold a first end of a tether 310. In the illustrated embodiment, tether attachment 308 is U-shaped and is attached to distal anchor 304 by inserting its free ends into distal end 304d and fixing them into the distal anchor. In one embodiment distal end 302d of support wire 302, and the free ends of tether attachment 308, can be attached to distal anchor 304 by welding, but other embodiments can use other types of attachments, such as soldering, brazing, adhesives, fasteners, etc. In various embodiments, distal end 302d and tether attachment 308 can be, but need not be, attached to distal anchor 304 by the same method.

Proximal anchor 306 has a distal end 306d, a proximal end 306p, and is spaced apart in a proximal direction from the distal anchor by a distance S between proximal end 304p and distal end 306d. A first hole 305 in proximal anchor 306 extends from distal end 306d to proximal end 306p to receive support wire 302 and fix it to the proximal anchor, so that the proximal anchor, like the distal anchor, is attached to the support wire and cannot move relative to the support wire.

A tethering mechanism is formed on proximal anchor 306 to work together with tether attachment 308. The tethering mechanism includes a notch 312 formed on a side of the proximal anchor. In the illustrated embodiment, notch 312 includes three substantially planar facets: a first facet 312a whose normal vector is substantially parallel to the support wire, a second facet 312b whose normal vector is substantially perpendicular to the support wire, and a third facet 312c whose normal vector is neither parallel nor perpendicular to the support wire. Other embodiments of notch 312 can be configured differently; for instance, in other embodiments notch 312 can be a two-facet notch, such as a simple V-notch, or can be a notch with both planar and curved surfaces, such as a U-notch. In still other embodiments, the tethering mechanism need not use a notch at all.

A second hole 314 is formed in proximal anchor 306. The second hole 314 can be substantially parallel to first hole 305. Second hole 314 extends through notch 312 and has two parts: one that extends from proximal end 306p to first facet 312a, and another that extends from third facet 312c to distal end 306d. Both parts of second hole 314 can slidably receive a release wire 316 - that is, the release wire is not fixed to the proximal anchor as is the support wire, but rather remains free to slide through second hole 314 relative to the proximal anchor in both distal and proximal directions.

Release wire 316 is first inserted into the first part of second hole 314. A second end of tether 310 is positioned in the notch, and the release wire is then threaded through the second end of tether 310 and into and through the second part of hole 314, so that release wire 316 completely spans notch 312. In this closed position, then, the first end of tether 310 is attached to tether attachment 308 and the second end of the tether is retained in notch 312 by the release wire. But because it is not fixed to proximal anchor 306, release wire 316 can be moved in a proximal direction from the closed position to an open position where it no longer spans all the way across notch 312. When release wire 316 slides to the open position, the second end of tether 310 can be released from the notch. In the illustrated embodiment, release wire 316 extends through the entire length of the proximal anchor and across the entire gap between proximal anchor 306 and distal anchor 304, so that its distal end 316d abuts proximal end 304p of the distal anchor. But in other embodiments, release wire 316 need not extend across this entire distance; in one embodiment, for instance, distal end 316d can be positioned at or about the distal end 306d of the proximal anchor.

Release wire 316 is moved from its closed position to its open position by pulling on its proximal end. In one embodiment, the proximal end of the release wire is positioned at or near the proximal end of a catheter (see, e.g., FIG. 2) so that a surgeon can use the release wire to release the tether when ready. Generally, it is desirable for the throw of the release wire-i.e., the distance between the closed position where it retains the tether and the open position where it releases the tether-to be large enough that the tether release will be perceptible to a surgeon using the apparatus. In the illustrated embodiment the throw is substantially the distance L between the proximal end 304p of the distal anchor and the third facet 312c or first facet 312a of notch 312, but in other embodiments the throw can be different than distance L. For example, the distance L may be between the proximal end 304 p and a location intermediate between the first facet 312a and the third facet 312c.

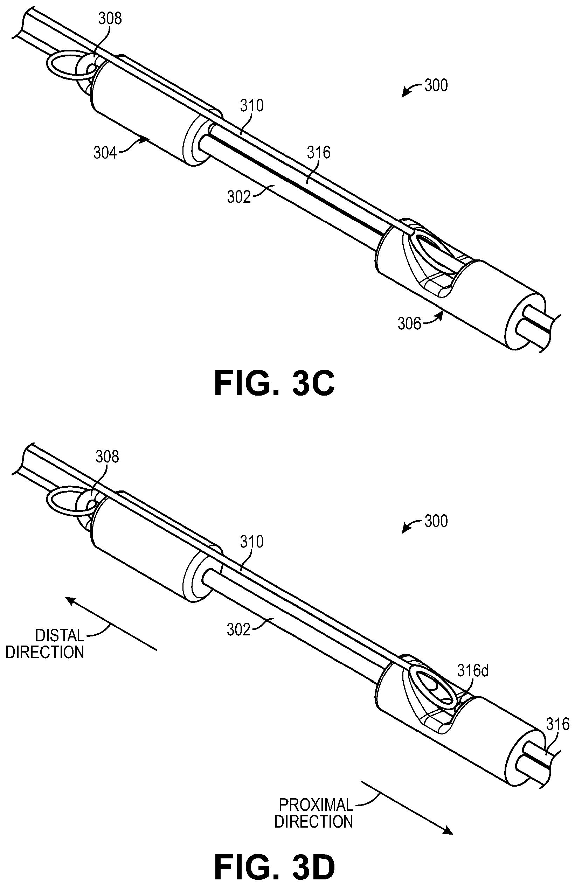

FIGS. 3C-3D together illustrate an embodiment of the operation of tethering apparatus 300. FIG. 3C illustrates the apparatus with release wire 316 in its closed or retaining position. In this position, the first end of tether 310 is attached to tether attachment 308 and the second end of the tether is retained in notch 312 by release wire 316.

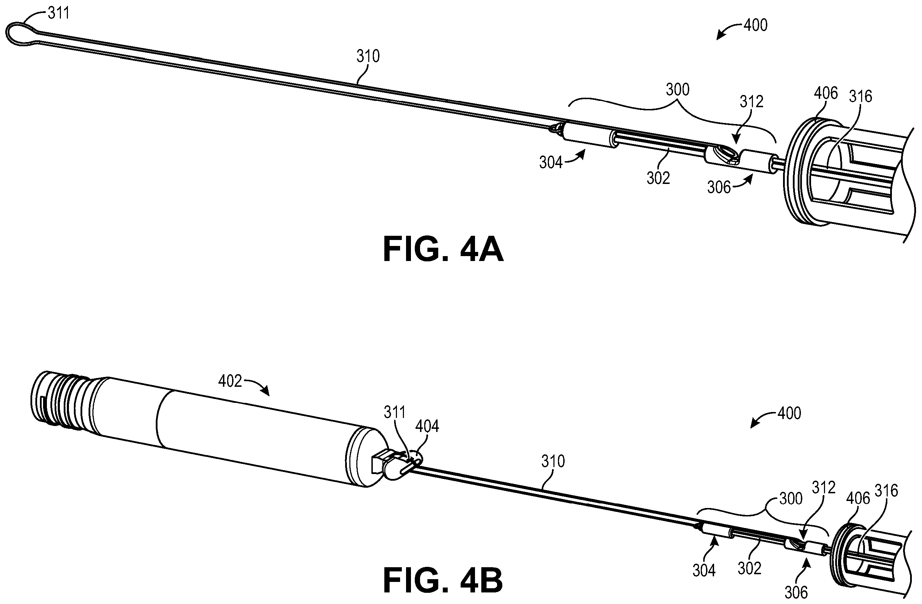

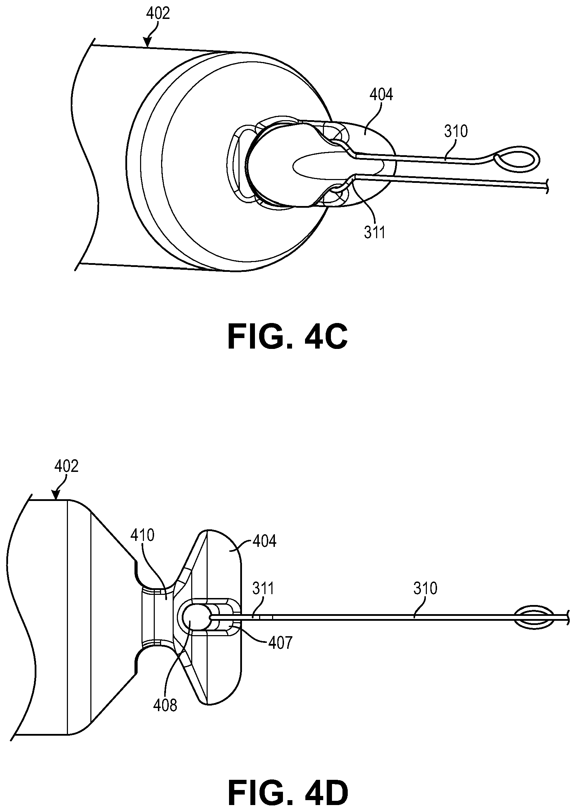

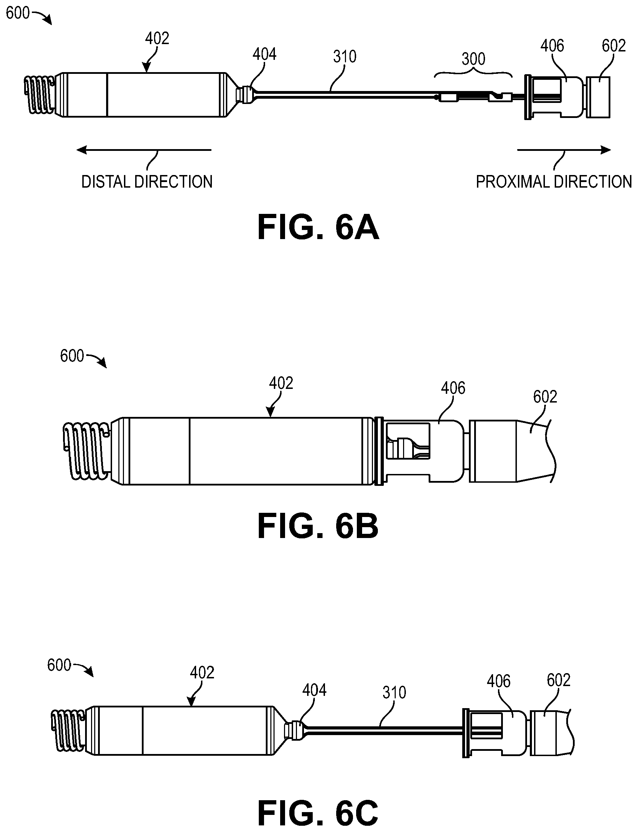

FIG. 3D illustrates the apparatus with release wire 316 in its open or release position. In the release position, release wire 316 has been moved in the proximal direction from the retaining position shown in FIG. 3C a sufficient distance (substantially distance L in the illustrated embodiment) so that it no longer spans the entire notch 312 and a second end of tether 310 can slip off the distal end 316d of the retaining wire and out of notch 312. When this happens, the second end of tether 310 is released from notch 312, while the first end of the tether remains attached to tether attachment 308. With tether 310 still attached to tether attachment 308, the tether can be pulled out of its attachment to a biostimulator (see, e.g., FIGS. 4A-4D and FIGS. 6A-6C) and the biostimulator is then left behind in a patient.

FIGS. 4A-4D together illustrate an embodiment of a biostimulator system 400 that uses a tethering apparatus such as tethering apparatus 300. FIGS. 4A-4B illustrate an embodiment of the attachment of tether 310 to a biostimulator 402, such as a leadless pacemaker. FIG. 4A illustrates the system 400 without biostimulator 402 attached to tether 310, and FIG. 4B illustrates the system with biostimulator 402 attached to the tether. In tethering apparatus 300, support wire 302 and release wire 316 have their distal ends at or near the distal end of a catheter and their proximal ends at or near the proximal end of the catheter (see, e.g., FIG. 2). As shown in FIG. 4A, tethering apparatus 300 is pulled out of an end cap 406 that is attached to the distal end of a catheter. Tether 310 can come with its first end pre-attached to tether attachment 308 or, in some embodiments, the first end of the tether can be attached to the tether attachment by a surgeon.

With the first end of the tether attached to tether attachment 308, a middle part 311 of tether 310 is attached to an attachment feature of the biostimulator 402, e.g., the attachment feature 404 of biostimulator 402, which is sometimes referred to as the "button" of the biostimulator. In one embodiment the middle part 311 of the tether can be approximately the middle of the tether, but in other embodiment it need not be exactly the middle. Also, in the illustrated embodiment a loop is formed in middle part 311, but other embodiments need not have a loop formed at middle part 311. After the middle part 311 of the tether is attached to button 404, the second end of the tether is inserted into notch 312 on proximal anchor 306 and retaining wire 316 is slid in a distal direction at least until it completely spans the notch, thereby securing the second end of the tether to the proximal anchor. In this arrangement, then, both ends of tether 310 attached to tether apparatus 300-the first end to tether attachment 308 and the second end to notch 312 and release wire 316.

FIGS. 4C-4D illustrate an embodiment of an biostimulator button 404 and how the middle part 311 of tether 310 can be attached to the button. Button 404 has an oblong shape and is attached to the main body of the biostimulator by a neck 410. A hole 408 is formed to receive tether 310. In the illustrated embodiment, hole 408 is formed where oblong shape 404 transitions to neck 410, but in other embodiments button 404 can be formed differently and hole 408 can be positioned differently, for instance entirely within oblong shape 404. Generally, it is desirable for hole 408 to have smooth edges so that tether 310 is easily inserted in, and easily pulled through and out of the hole without catching on any sharp corners. In the illustrated embodiment, hole 408 has a smooth transition zone 407 in at least the direction of the tether, but other embodiments of hole 406 can have more, less, or different smooth edges than shown.

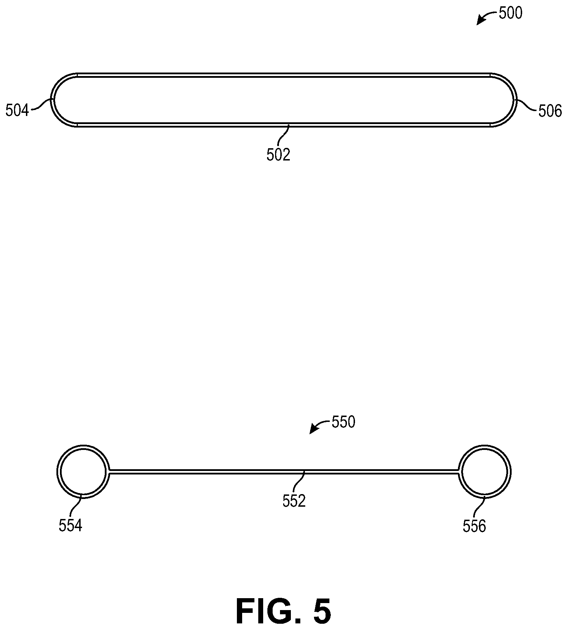

FIG. 5 illustrates a pair of embodiments of flexible tethers 500 and 550 that can be used with tethering apparatus 300. Tether 500 includes a strand 502 of a flexible fiber formed into a single loop, so that the loop has a first end 504 and a second end 506. Tether 550 includes a strand 552 of flexible fiber, with a loop 554 formed at a first end of strand 552 and another loop 556 formed at a second end of the strand. In the illustrated embodiment of tether 550 both loops 554 and 556 are circular and of substantially the same size, but in other embodiments they can have a different shape, such as elliptical, and in still other embodiments loops 554 and 556 need not have the same shape or size.

In some embodiments, tethers 500 and 550 can be made of a fibrous, polymeric, non-metallic material. For instance, in one embodiment the tethers can be made from commonly available surgical suture materials such as Ultra High Molecular Weight Polyethylene (UHMWPE) and Polyester (PE). Other embodiments can use other materials, such as Kevlar^{®}, Vectran^{®}, etc. These materials offer benefits including high tensile strength, being very flexible and supple, virtually infinite fatigue life, and low cost.

FIGS. 6A-6C illustrate an embodiment of the operation of a biostimulator delivery system 600 including a catheter, a tethering apparatus 300, and a biostimulator. FIG. 6A illustrates system 600 in preparation mode, i.e., when it is being prepared to implant biostimulator 402. In this mode, the support and release wires are pulled or pushed in a distal direction until tethering apparatus 300 emerges from the distal end of catheter 602 and moves beyond end cap 406, which is attached to the distal end of catheter 602. This exposes the tethering apparatus so that the surgeon can attach the tether 310. The tethering apparatus can come with a first end of tether 310 pre-attached to tether attachment 308, or the surgeon can attach the first end to the tether attachment. After the first end of the tether is secured to the tether attachment, the surgeon then routes the second end of tether 310 from tether attachment 308, through button 404, and back to the tethering mechanism on proximal anchor 306. The second end of tether 310 is inserted into the notch on proximal anchor 306 and the release wire 316 is then slid in a distal direction until it spans the notch 312, at which point the second end of tether 310 is secured in the proximal anchor.

FIG. 6B illustrates system 600 in a state where it is ready for insertion of the biostimulator 402 into a patient. In this state, once the tether is attached to both the biostimulator 402 and the tethering apparatus 300, as shown in FIG. 6A, the support wire and the retention wire are pulled or pushed in a proximal direction, thus pulling tethering apparatus 300 and tether 310 through the distal end of catheter 602 and into the interior of the catheter. The support wire and the retention wire are pulled in a proximal direction until button 404, and thus biostimulator 402, are engaged by end cap 406. The end cap is positioned at the distal end of the catheter and is essentially a socket that engages and releases the biostimulator and allows the catheter to exert forces and torques on the biostimulator during implantation.

FIG. 6C illustrates system 600 in tether mode. In tether mode, biostimulator 402 is disengaged from end cap 406 but remains attached to tether 310, so that the mechanical connection of the biostimulator to the heart tissue where it was implanted can be evaluated before the biostimulator is completely released. In the illustrated embodiment, tethering apparatus 300 remains within catheter 602 during tether mode, but in other embodiments the tethering apparatus can be moved outside the catheter during tether mode by pushing the proximal ends of the support and release wires in the distal direction until the tethering apparatus emerges from the distal end of the catheter.

If, while in tether mode, the biostimulator 402 is found not to be satisfactorily attached to the heart tissue, then tether 310 can be used to pull biostimulator 402 back into engagement with end cap 406 by pulling the support wire in a proximal direction without releasing the tether from the proximal and distal anchors. But if biostimulator 402 is satisfactorily attached to the heart tissue, tether 310 can then be removed from the biostimulator. To remove the tether from the biostimulator, the second end of the tether is released from proximal anchor 306 by moving release wire 316 in the proximal direction until it releases (i.e., no longer engages) the second end of the tether. Once the second end is released from the notch 312, the support wire 302 can then be pulled in a proximal direction. Because the first end of the tether 310 remains attached to tether attachment 308 on distal anchor 304, pulling the support wire 302 in a proximal direction also pulls the tether 310 in a proximal direction, causing the second end of the tether 310 to be pulled through hole 406 on button 404 and thus releasing the biostimulator from the tether.

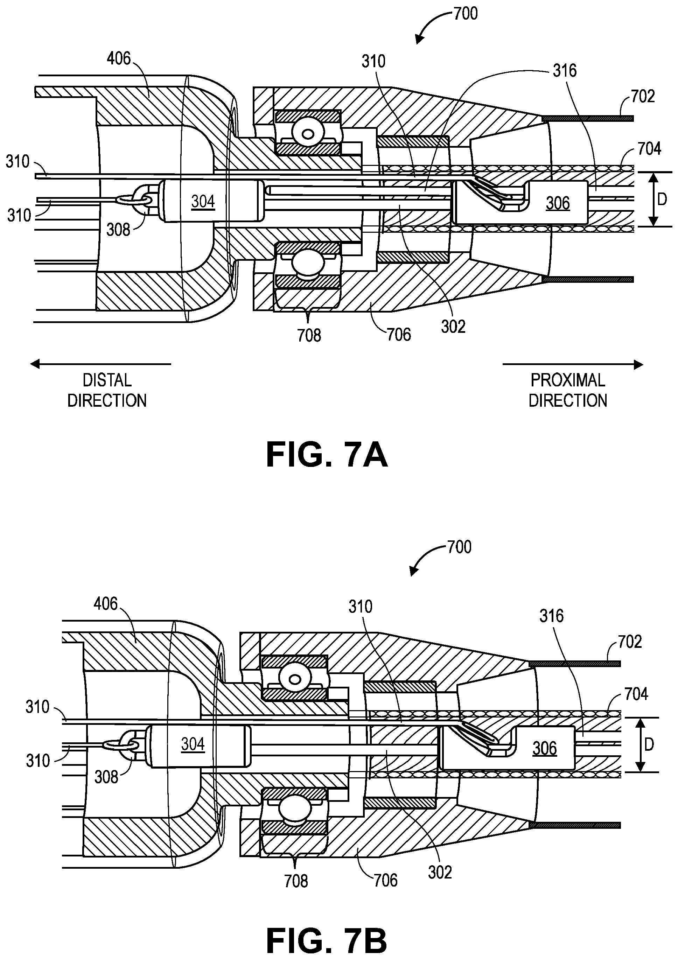

FIGS. 7A-7B together illustrate an embodiment of a catheter 700 with a tether mechanism 300 inside. Catheter 700 includes an outer sheath 702 with a torque shaft 704 inside. The catheter can include additional components between sheath 702 and torque shaft 704, but they are not shown in these drawings. An end socket 706 is attached to the distal end of the sheath and torque shaft, and the end socket includes a locking mechanism use to firmly secure end cap 406 to the distal end of the catheter. As discussed above, end cap 406 is essentially a socket that engages and releases the biostimulator and allows the catheter, primarily through torque shaft 704, to exert forces and torques on the biostimulator during implantation.

In the illustrated embodiment, the components of tethering apparatus 300, when pulled inside the catheter, reside within torque shaft 704. As described above, the main components of the tethering apparatus 300 include support wire 302, distal anchor 304, proximal anchor 306, and release wire 316. Torque shaft 704 has an inner diameter, D, large enough to accommodate the tethering apparatus inside, and also large enough to allow the tethering apparatus to operate while inside the torque shaft. In other words, the diameter D of torque shaft 704 is large enough that, when the second end of tether 310 is released from proximal anchor 306, the second end of the tether can move in a distal direction through the torque shaft and the end cap until it is free of the catheter and can be pulled out of button 404 (see FIGS. 4A-4D).

The above description of embodiments is not intended to be exhaustive or to limit the invention to the described forms. Specific embodiments of, and examples for, the invention are described herein for illustrative purposes, but various modifications are possible.

## Claims

1. A biostimulator transport system (202), comprising:
a distal anchor (304) including a tether attachment (308) adapted to secure a first end of a tether (310);
a proximal anchor (306); and
a tethering mechanism coupled to the proximal anchor (306), wherein the tethering mechanism is adapted to receive a second end of the tether (310), and wherein the tethering mechanism is movable between a closed position that retains the second end of the tether (310) and an open position that releases the second end of the tether (310), **characterized by** a support wire (302), wherein
the distal anchor (304) is attached to the support wire (302); and
the proximal anchor (306) is attached to the support wire (302) proximal to the distal anchor (304).

2. The biostimulator transport system of claim 1, wherein a hole (314) extends through the proximal anchor (306), and wherein the tethering mechanism comprises:
a notch (312) formed in the proximal anchor (306); and
a release wire (316) slidably positioned in the hole (314), wherein the release wire (316) is movable between the closed position where it fully spans the notch (312) and the open position where it does not fully span the notch (312).

3. The biostimulator transport system of claim 2, wherein the notch (312) includes a first facet (312a) whose normal vector is substantially parallel to the support wire (302), a second facet (312b) whose normal vector is substantially perpendicular to the support wire (302), and a third facet (312c) whose normal vector is oblique to the support wire (302).

4. The biostimulator transport system of claim 3, wherein the hole (314) has a first part that extends through the proximal anchor (306) to the third facet (312c) and a second part that extends through the proximal anchor (306) to the first facet (312a).

5. The biostimulator transport system of any one of claims 2 to 4, wherein, when the release wire (316) is in the closed position, the release wire (316) is in contact with the distal anchor (304).

6. The biostimulator transport system of any one of claims 1 to 5, further comprising the tether (310) having the first end and the second end.

7. The biostimulator transport system of claim 6, wherein the first end of the tether (310) is permanently connected to the tether attachment.

8. The biostimulator transport system of claim 6 or 7, wherein a biostimulator (100, 402) is coupled to the tether (310) between the first end and the second end.

9. The biostimulator transport system of any one of claims 1 to 8, further comprising:
a catheter having an end cap (406) coupled to a distal catheter end; and
a tethering apparatus (300) positioned within the catheter and movable relative to the catheter, the tethering apparatus (300) comprising the support wire (302), the distal anchor (304), the proximal anchor (306), and the tethering mechanism.

10. The biostimulator transport system of claim 9, wherein the tethering apparatus (300) is movable relative to the catheter between a position outside the catheter and a position inside the catheter.

11. The biostimulator transport system of claim 10, wherein the catheter includes a torque shaft (704), and wherein, when inside the catheter, the tethering mechanism is inside the torque shaft (704).

12. The biostimulator transport system of claim 11, wherein the tethering mechanism is operable when the tethering apparatus (300) is inside the catheter and when the tethering apparatus (300) is outside the catheter.

13. The biostimulator transport system of any one of claims 9 to 12, wherein the tether (310) has the first end coupled to the tether attachment, the second end releasably coupled to the tethering mechanism, and a middle portion coupled to a biostimulator (100, 402) releasably engaged in the end cap (406).

14. The biostimulator system of any one of claims 2 to 5 in combination with any one of claims 9 to 13, wherein a proximal end of the support wire (302) and a proximal end of the release wire (316) are located at a proximal end of the catheter.

15. The biostimulator system of any one of claims 1 to 14, wherein the tether (310) includes a fiber tether (310).

## Patentansprüche

1. Biostimulatortransportsystem (202), umfassend:
einen distalen Anker (304), der eine Halteseilbefestigung (308) beinhaltet, die dazu ausgelegt ist, ein erstes Ende eines Halteseils (310) zu sichern;
einen proximalen Anker (306); und
einen Haltemechanismus, der an den proximalen Anker (306) gekoppelt ist, wobei der Haltemechanismus dazu ausgelegt ist, ein zweites Ende des Halteseils (310) aufzunehmen, und wobei der Haltemechanismus zwischen einer geschlossenen Position, die das zweite Ende des Halteseils (310) hält, und einer offenen Position, die das zweite Ende des Halteseils (310) freigibt, bewegbar ist, **gekennzeichnet durch** einen Stützdraht (302), wobei der distale Anker (304) an dem Stützdraht (302) befestigt ist; und
der proximale Anker (306) proximal zu dem distalen Anker (304) an dem Stützdraht (302) befestigt ist.

2. Biostimulatortransportsystem nach Anspruch 1, wobei sich ein Loch (314) durch den proximalen Anker (306) erstreckt und wobei der Haltemechanismus Folgendes umfasst:
eine Kerbe (312), die in dem proximalen Anker (306) ausgebildet ist; und
einen Freigabedraht (316), der verschiebbar in dem Loch (314) positioniert ist, wobei der Freigabedraht (316) zwischen der geschlossenen Position, in der er die Kerbe (312) vollständig überspannt, und der offenen Position, in der er die Kerbe (312) nicht vollständig überspannt, bewegbar ist.

3. Biostimulatortransportsystem nach Anspruch 2, wobei die Kerbe (312) eine erste Facette (312a), deren Normalvektor im Wesentlichen parallel zu dem Stützdraht (302) ist, eine zweite Facette (312b), deren Normalvektor im Wesentlichen senkrecht zu dem Stützdraht (302) ist, und eine dritte Facette (312c), deren Normalvektor schräg zu dem Stützdraht (302) ist, beinhaltet.

4. Biostimulatortransportsystem nach Anspruch 3, wobei das Loch (314) einen ersten Teil, der sich durch den proximalen Anker (306) zu der dritten Facette (312c) erstreckt, und einen zweiten Teil, der sich durch den proximalen Anker (306) zu der ersten Facette (312a) erstreckt, aufweist.

5. Biostimulatortransportsystem nach einem der Ansprüche 2 bis 4, wobei der Freigabedraht (316), wenn sich der Freigabedraht (316) in der geschlossenen Position befindet, mit dem distalen Anker (304) in Kontakt steht.

6. Biostimulatortransportsystem nach einem der Ansprüche 1 bis 5, ferner umfassend das Halteseil (310), das das erste Ende und das zweite Ende aufweist.

7. Biostimulatortransportsystem nach Anspruch 6, wobei das erste Ende des Halteseils (310) dauerhaft mit der Halteseilbefestigung verbunden ist.

8. Biostimulatortransportsystem nach Anspruch 6 oder 7, wobei ein Biostimulator (100, 402) zwischen dem ersten Ende und dem zweiten Ende an das Halteseil (310) gekoppelt ist.

9. Biostimulatortransportsystem nach einem der Ansprüche 1 bis 8, ferner umfassend:
einen Katheter, der eine Endkappe (406) aufweist, die an ein distales Katheterende gekoppelt ist; und
eine Haltevorrichtung (300), die innerhalb des Katheters positioniert und relativ zu dem Katheter bewegbar ist, wobei die Haltevorrichtung (300) den Stützdraht (302), den distalen Anker (304), den proximalen Anker (306) und den Haltemechanismus umfasst.

10. Biostimulatortransportsystem nach Anspruch 9, wobei die Haltevorrichtung (300) relativ zu dem Katheter zwischen einer Position außerhalb des Katheters und einer Position innerhalb des Katheters bewegbar ist.

11. Biostimulatortransportsystem nach Anspruch 10, wobei der Katheter eine Drehmomentwelle (704) beinhaltet und wobei sich der Haltemechanismus, wenn er sich innerhalb des Katheters befindet, innerhalb der Drehmomentwelle (704) befindet.

12. Biostimulatortransportsystem nach Anspruch 11, wobei der Haltemechanismus betreibbar ist, wenn sich die Haltevorrichtung (300) innerhalb des Katheters befindet und wenn sich die Haltevorrichtung (300) außerhalb des Katheters befindet.

13. Biostimulatortransportsystem nach einem der Ansprüche 9 bis 12, wobei das erste Ende des Halteseils (310) mit der Halteseilbefestigung gekoppelt ist, das zweite Ende lösbar mit dem Haltemechanismus gekoppelt ist und ein mittlerer Abschnitt mit einem Biostimulator (100, 402) gekoppelt ist, der lösbar in der Endkappe (406) in Eingriff steht.

14. Biostimulatorsystem nach einem der Ansprüche 2 bis 5 in Kombination mit einem der Ansprüche 9 bis 13, wobei sich ein proximales Ende des Stützdrahts (302) und ein proximales Ende des Freigabedrahts (316) an einem proximalen Ende des Katheters befinden.

15. Biostimulatorsystem nach einem der Ansprüche 1 bis 14, wobei das Halteseil (310) ein Faserhalteseil (310) beinhaltet.

## Revendications

1. Système de transport de biostimulateur (202), comprenant :
un ancrage distal (304) comprenant une fixation d'une attache (308) conçue pour fixer une première extrémité d'une attache (310) ;
un ancrage proximal (306) ; et
un mécanisme d'attache couplé à l'ancrage proximal (306), dans lequel le mécanisme d'attache est conçu pour recevoir une seconde extrémité de l'attache (310), et dans lequel le mécanisme d'attache est mobile entre une position fermée qui retient la seconde extrémité de l'attache (310) et une position ouverte qui libère la seconde extrémité de l'attache (310), **caractérisé par** un fil de support (302), dans lequel l'ancrage distal (304) est fixé au fil de support (302) ; et l'ancrage proximal (306) est fixé au fil de support (302) en position proximale par rapport à l'ancrage distal (304).

2. Système de transport de biostimulateur selon la revendication 1, dans lequel un trou (314) s'étend à travers l'ancrage proximal (306), et dans lequel le mécanisme d'attache comprend :
une encoche (312) formée dans l'ancrage proximal (306) ; et
un fil de libération (316) positionné de manière coulissante dans le trou (314), dans lequel le fil de libération (316) est mobile entre la position fermée où il enjambe entièrement l'encoche (312) et la position ouverte où il n'enjambe pas entièrement l'encoche (312).

3. Système de transport de biostimulateur selon la revendication 2, dans lequel l'encoche (312) comprend une première facette (312a) dont le vecteur normal est sensiblement parallèle au fil de support (302), une deuxième facette (312b) dont le vecteur normal est sensiblement perpendiculaire au fil de support (302), et une troisième facette (312c) dont le vecteur normal est oblique par rapport au fil de support (302).

4. Système de transport de biostimulateur selon la revendication 3, dans lequel le trou (314) présente une première partie qui s'étend à travers l'ancrage proximal (306) jusqu'à la troisième facette (312c) et une seconde partie qui s'étend à travers l'ancrage proximal (306) jusqu'à la première facette (312a).

5. Système de transport de biostimulateur selon l'une quelconque des revendications 2 à 4, dans lequel, lorsque le fil de libération (316) se trouve dans la position fermée, le fil de libération (316) est en contact avec l'ancrage distal (304).

6. Système de transport de biostimulateur selon l'une quelconque des revendications 1 à 5, comprenant en outre l'attache (310) ayant la première extrémité et la seconde extrémité.

7. Système de transport de biostimulateur selon la revendication 6, dans lequel la première extrémité de l'attache (310) est raccordée de manière permanente à la fixation de l'attache.

8. Système de transport de biostimulateur selon la revendication 6 ou 7, dans lequel un biostimulateur (100, 402) est couplé à l'attache (310) entre la première extrémité et la seconde extrémité.

9. Système de transport de biostimulateur selon l'une quelconque des revendications 1 à 8, comprenant en outre :
un cathéter ayant un embout (406) couplé à une extrémité distale de cathéter ; et
un appareil d'attache (300) positionné à l'intérieur du cathéter et mobile par rapport au cathéter, l'appareil d'attache (300) comprenant le fil de support (302), l'ancrage distal (304),
l'ancrage proximal (306) et le mécanisme d'attache.

10. Système de transport de biostimulateur selon la revendication 9, dans lequel l'appareil d'attache (300) est mobile par rapport au cathéter entre une position à l'extérieur du cathéter et une position à l'intérieur du cathéter.

11. Système de transport de biostimulateur selon la revendication 10, dans lequel le cathéter comprend un arbre de transmission de couple (704), et dans lequel, lorsqu'il se trouve à l'intérieur du cathéter, le mécanisme d'attache se trouve à l'intérieur de l'arbre de transmission de couple (704).

12. Système de transport de biostimulateur selon la revendication 11, dans lequel le mécanisme d'attache est actionnable lorsque l'appareil d'attache (300) se trouve à l'intérieur du cathéter et lorsque l'appareil d'attache (300) se trouve à l'extérieur du cathéter.

13. Système de transport de biostimulateur selon l'une quelconque des revendications 9 à 12, dans lequel l'attache (310) a la première extrémité couplée à la fixation de l'attache, la seconde extrémité couplée de manière libérable au mécanisme d'attache, et une partie médiane couplée à un biostimulateur (100, 402) en prise de manière libérable dans l'embout (406).

14. Système de biostimulateur selon l'une quelconque des revendications 2 à 5 en combinaison avec l'une quelconque des revendications 9 à 13, dans lequel une extrémité proximale du fil de support (302) et une extrémité proximale du fil de libération (316) sont situées au niveau d'une extrémité proximale du cathéter.

15. Système de biostimulateur selon l'une quelconque des revendications 1 à 14, dans lequel l'attache (310) comprend une attache en fibre (310).
